Europäisches Patentamt

European Patent Office

Office européen des brevets

(19)

(11) Publication number: **0 091 527**
**A2**

(12) # EUROPEAN PATENT APPLICATION

(21) Application number: **82306643.6**

(22) Date of filing: **13.12.82**

(51) Int. Cl.³: **C 12 N 15/00**
C 12 P 21/02, C 12 N 1/20
C 07 C 103/52, C 07 H 21/04
A 61 K 37/02
//C12R1/19, C12R1/38, C12R1/07

(30) Priority: **14.12.81 US 330912**

(43) Date of publication of application:
**19.10.83 Bulletin 83/42**

(84) Designated Contracting States:
**AT BE CH DE FR GB IT LI LU NL SE**

(71) Applicant: **PRESIDENT AND FELLOWS OF HARVARD COLLEGE**
**17 Quincy Street**
**Cambridge Massachusetts 02138(US)**

(72) Inventor: **Gilbert, Walter**
**107 Upland Road**
**Cambridge Massachusetts 02140(US)**

(72) Inventor: **Philipp, Barbara Wallner**
**32 Rockledge Road**
**Newton Massachusetts 02161(US)**

(74) Representative: **Bannerman, David Gardner et al,**
**Withers & Rogers 4 Dyer's Buildings Holborn**
**London, EC1N 2JT(GB)**

(54) DNA sequences, recombinant DNA molecules and processes for producing human serum albumin-like polypeptides.

(57) DNA sequences, recombinant DNA molecules and processes for producing human serum albumin-like polypeptides. The DNA sequences and recombinant DNA molecules of this invention are characterized in that they include DNA fragments that code for human serum albumin-like polypeptides. These DNA sequences and recombinant DNA molecules and the hosts transformed with them may be employed in the processes of this invention to produce human serum albumin-like polypeptides.

EP 0 091 527 A2

DGB/JEA                    -1-

# DNA SEQUENCES, RECOMBINANT DNA MOLECULES AND PROCESSES FOR PRODUCING HUMAN SERUM ALBUMIN-LIKE POLYPEPTIDES

## BACKGROUND OF THE INVENTION

This invention relates to DNA sequences, recombinant DNA molecules and processes for producing human serum albumin-like polypeptides. More particularly, the invention relates to DNA sequences and recombinant DNA molecules expressed in appropriate host organisms.

The DNA sequences and recombinant DNA molecules of this invention are characterized in that they include fragments that code for human serum albumin-like polypeptides. Accordingly, these DNA sequences and recombinant DNA molecules and the hosts transformed with them may be employed in the processes of this invention to produce human serum albumin-like polypeptides.

Human serum albumin is a major protein component of human serum. It is synthesized by the liver. It appears to control the osmotic pressure of the intravascular fluid and to bind a variety of metabolites in the blood. The amino acid sequence of natural human serum albumin is known [B. Meloun et al., FEBS Letters, 58, pp. 134-37 (October 1975)]. It is a protein of 585 amino acids.

Today, human serum albumin is prepared by isolating it from blood samples that are no longer useful for transfusions. It has found widespread application as a blood supplement for burn patients, as a means to improve the performance of the human circulatory system, as an amino acid source for food additives, as a potential nitrogen fixer, in the treatment of kernicterus (excess of bilirubin) in newborn infants and as a pharmaceutically-acceptable carrier for many drugs and other compounds employed for human therapy.

Since human serum albumin is purified from blood samples, it is susceptible to the same contamination as blood. For example, human serum albumin may be contaminated with hepatitis B virus particles or other viral and toxic substances that may be transmitted among humans by blood transfusion. Plainly, the possibility of such contamination has limited the use of human serum albumin. Moreover, since its major source is about-to-be-discarded blood samples, the supply of human serum albumin will decrease as blood storage conditions improve. A similar decrease in the availability of human serum albumin will also occur as human serum albumin finds more widespread use in pharmaceutical therapy. Accordingly, other sources of highly-purified human serum albumin are required.

Recent advances in molecular biology have made it possible to produce large amounts of eukaryotic proteins in bacterial hosts. These include, for example, leukocyte interferon (S. Nagata et al., "Synthesis In E. coli Of A Polypeptide With Human Leukocyte Interferon Activity", Nature, 284, pp. 316-20 (1980)), antigens of human hepatitis B virus (C. J. Burrell et al., "Expression In Escherichia coli Of Hepatitis B Virus DNA Sequences Cloned In Plasmid pBR322", Nature, 279, pp. 43-7 (1979) and M. Pasek et al., "Hepatitis B Virus Genes And Their Expression In E. coli", Nature, 282, pp. 575-79 (1979)), SV40t antigen (T. M. Roberts et al., "Synthesis Of Simian Virus 40t Antigen In Escherichia coli", Proc. Natl. Acad. Sci. USA, 76, pp. 5596-5600 (1979)), and FMD viral antigens (H. Küpper et al., "Cloning Of cDNA Of Major Antigen Of Foot And Mouth Disease Virus And Expression In E. coli", Nature, 289, pp. 555-59 (1982)).

In general, these processes rely on the construction of recombinant DNA molecules characterized by a DNA

0091527

-3-

sequence coding for the desired product operatively linked to an expression control sequence. Appropriate hosts are then transformed with these molecules to permit production of the desired product by fermentation processes. For DNA coding sequences, other than those prepared via chemical synthesis, the construction of such recombinant DNA molecules comprises the steps of producing a single-stranded DNA copy (cDNA) of a messenger RNA (mRNA) template for the desired protein; converting the cDNA to double-stranded DNA and operatively linking the DNA to an appropriate expression control sequence in an appropriate cloning vehicle. The recombinant DNA molecule is then employed to transform an appropriate host. Such transformation may permit that host to produce the desired protein when it is fermented under appropriate conditions.

## SUMMARY OF THE INVENTION

The present invention provides at least one DNA sequence coding for a human serum albumin-like polypeptide. More particularly, we provide in accordance with this invention a DNA sequence characterized in that at least a portion thereof codes for a human serum albumin-like polypeptide. The DNA sequences of this invention are selected from the group consisting of (a) HSA/33-1, HSA/17-3, HSA/33-1(BglII-EcoRI)-HSA/17-3(BglII-EcoRI), HSA/33-1 (XbaI-EcoRI)-HSA/17-3(XbaI-EcoRI) (b) DNA sequences which hybridize to any of the foregoing DNA sequences and which code for a human serum albumin-like polypeptide; (c) DNA sequences, from whatever source obtained including natural, synthetic or semisynthetic sources, related by mutation, including single or multiple, base substitutions, deletions, insertions and inversions to any of the foregoing DNA sequences and which code for a human serum albumin-like polypeptide and (d) DNA sequences comprising sequences of codons which code for a polypeptide containing an amino acid sequence similar to those coded for by the codons of any of the foregoing DNA sequences and which code for human serum albumin-like polypeptide.

By virtue of this invention, it is accordingly possible to obtain human serum albumin-like polypeptides in substantial quantities and in a form that cannot possibly be contaminated with hepatitis B virus particles or other impurities formerly inherent in natural human serum albumin isolated from human blood and plasma. The DNA sequences, recombinant DNA molecules, hosts and processes of using them and the human serum albumin-like polypeptides produced by them in this invention avoid the problems which have beset the other known methods of human serum albumin production. Accordingly, they enable large amounts of highly-pure human serum albumin-like polypeptides and their derivatives to be made available for diverse uses in the pharmaceutical and other industries.

## BRIEF DESCRIPTION OF THE DRAWINGS

Figure 1 is a schematic outline of one embodiment of a process of this invention for producing the human serum albumin-like polypeptides of this invention.

Figure 2 is a partial restriction map of three DNA sequences and recombinant DNA molecules of this invention: pKT218(HSA/33-1), pKT218(HSA/17-3) and pKT218 (HSA/33-1(BglII-EcoRI)-HSA/17-3(BglII-EcoRI)) and displays the combination of the first two sequences to produce the third.

Figure 3 displays a partial restriction map of DNA sequence HSA/33-1(BglII-EcoRI)-HSA/17-3(BglII-EcoRI) and the strategy employed to determine portions of its nucleotide sequence. The distances indictated are approximate. They may be confirmed by nucleotide sequencing.

Figure 4 displays portions of the nucleotide sequence of HSA/33-1(BglII-EcoRI)-HSA/17-3(BglII-EcoRI) and the amino acid sequence of the coding regions of those portions.

Figure 5 displays a schematic outline of a process of preparing another recombinant DNA molecule of this invention.

Figure 6 displays a schematic outline of a process of preparing another recombinant DNA molecule of this invention.

Figure 7 displays a schematic outline of a process for preparing another recombinant DNA molecule of this invention.

Figure 8 displays a schematic outline of a process for preparing another recombinant DNA molecule of this invention.

DETAILED DESCRIPTION OF THE INVENTION

In order that the invention herein described may be more fully understood, the following detailed description is set forth.

In the description the following terms are employed:

Nucleotide - A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C and uracil ("U").

DNA Sequence - A linear array of nucleotides connected one to the other by phosphodiester bonds between the 3' and 5' carbons of adjacent pentoses.

Codon - A DNA sequence of three nucleotides (a triplet) which encodes through mRNA an amino acid, a translation start signal or a translation termination signal. For example, the nucleotide triplets TTA, TTG, CTT, CTC, CTA and CTG encode for the amino acid leucine ("Leu"), TAG, TAA and TGA are translation stop signals and ATG is a translation start signal.

Reading Frame - The grouping of codons during translation of mRNA into amino acid sequences. During translation the proper reading frame must be maintained.

For example, the sequence GCTGGTTGTAAG may be translated in three reading frames or phases, each of which affords a different amino acid sequence

GCT GGT TGT AAG -- Ala-Gly-Cys-Lys

G CTG GTT GTA AG -- Leu-Val-Val

GC TGG TTG TAA G -- Trp-Leu-(STOP) Polypeptide - A linear array of amino acids connected one to the other by peptide bonds between the α-amino and carboxy groups of adjacent amino acids.

Genome - The entire DNA of a cell or a virus. It includes inter alia the structural genes coding for the polypeptides of the cell or virus, as well as its operator, promoter and ribosome binding and interaction sequences, including sequences such as the Shine-Dalgarno sequences.

Structural Gene - A DNA sequence which encodes through its template or messenger RNA ("mRNA") a sequence of amino acids characteristic of a specific polypeptide.

Transcription - The process of producing mRNA from a structural gene.

Translation - The process of producing a polypeptide from mRNA.

Expression - The process undergone by a structural gene to produce a polypeptide. It is a combination of transcription and translation.

Plasmid - A non-chromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance (Tet$^R$) transforms a cell previously sensitive to tetracycline into one which is resistant to it. A cell transformed by a plasmid is called a "transformant".

Phage or Bacteriophage - Bacterial virus, many of which consist of DNA sequences encapsidated in a protein envelope or coat ("capsid protein").

Cloning Vehicle - A plasmid, phage DNA or other DNA sequence which is able to replicate in a host cell, which is characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance. A cloning vehicle is often called a vector.

Cloning - The process of obtaining a population of organisms or DNA sequences derived from one such organism or sequence by asexual reproduction.

Recombinant DNA Molecule or Hybrid DNA - A molecule consisting of segments of DNA from different genomes which have been joined end-to-end outside of living cells and have the capacity to infect some host cell and be maintained therein.

Expression Control Sequence - A sequence of nucleotides that controls and regulates expression of structural genes when operatively linked to those genes. They include the lac system, the trp system, the TAC system, the β-lac system, major operator and promoter regions of phage λ, the control region of fd coat protein and other sequences known to control the expression of genes of prokaryotic or eukaryotic cells or their viruses and various combinations of them.

Human Serum Albumin-Like Polypeptides - A polypeptide displaying a biological or immunological activity of natural human serum albumin ("HSA"). This polypeptide may contain amino acids which are not part of natural HSA or may contain only a portion of the amino acids of natural HSA. The polypeptide may also not be identical to natural HSA because the host in which it is made may lack appropriate enzymes which may be required to transform the host-produced polypeptide to the structure and substitution of natural HSA.

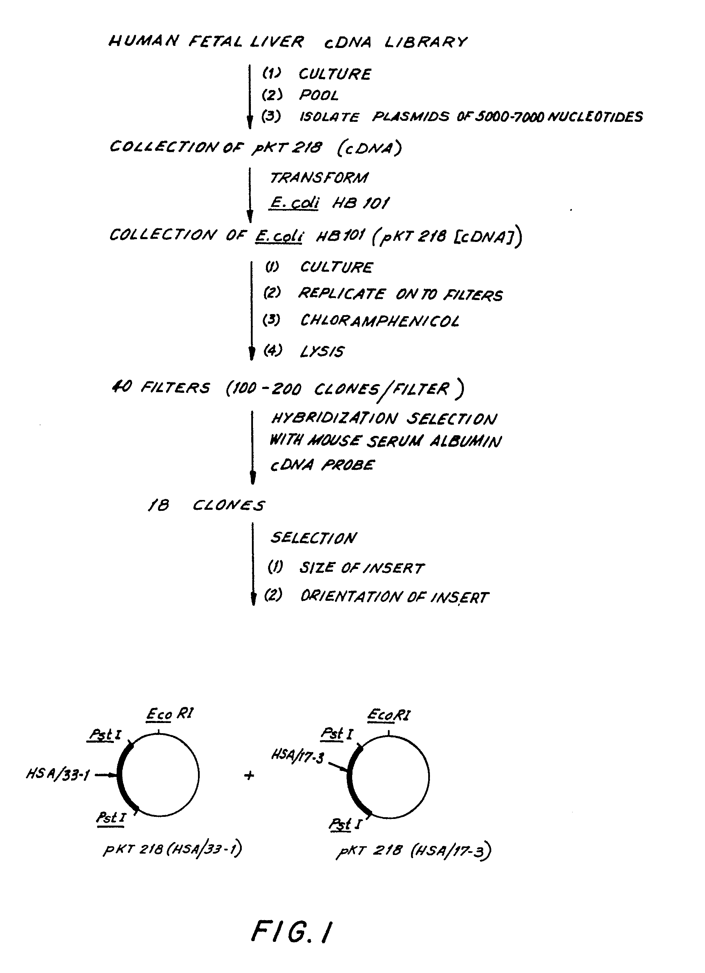

Referring now to Figure 1, we have shown therein a schematic outline of one embodiment of a process for preparing a DNA sequence and a recombinant DNA molecule of this invention.

<div align="center">EXAMPLE</div>

<div align="center">PREPARATION OF A HUMAN FETAL LIVER cDNA LIBRARY</div>

A human fetal liver cDNA library was prepared by David Kurnit of Harvard Medical School. PolyA RNA was isolated from human fetal liver using well-known methods. That polyA RNA was then used as a template to prepare single-stranded complementary DNA (cDNA) [e.g., A. Efstratiadis et al., "Full Length And Discrete Partial Reverse Transcripts Of Globin And Chorion mRNAs", Cell, 4, pp. 367-78 (1975) and references cited therein]. The cDNA was then rendered double-stranded by conventional methods, tailed with dC residues and inserted into the dG-tailed PstI site of pKT218 (a derivative of pBR322) [e.g. L. Villa-Komaroff et al., "A Bacterial Clone Synthesizing Proinsulin", Proc. Natl. Acad. Sci. USA, 75, pp. 3727-31 (1978); K. Talmadge et al., "Eukaryotic Signal Sequence Transports Insulin Antigen In Escherichia coli", Proc. Natl. Acad. Sci. USA, 77, pp. 3369-73 (1980)]. The resulting recombinant DNA molecules, comprising the human fetal liver library, were then employed to transform competent E. coli HB101 using standard procedures. Dr. Kurnit graciously made this library available to us.

<div align="center">SCREENING OF THE HUMAN FETAL LIVER cDNA
LIBRARY WITH MOUSE SERUM ALBUMIN cDNA</div>

We decided to screen the above-described human fetal liver cDNA library with mouse serum albumin cDNA [D. Kioussis et al., "The Evolution Of α-Fetoprotein And Albumin", Journal of Biological Chemistry, 256, pp. 1960-67 (February 25, 1981)]. The basis for our approach was that bovine and rat serum albumins have some amino acid sequence similarity to human serum albumin [T. Peters, Jr., "Serum Albumin" in The Plasma Proteins (F. W. Putnam ed.), vol. 1,

pp. 133-181 (1975)]. Accordingly, we postulated that mouse serum albumin cDNA might cross hybridize to human serum albumin cDNA to an extent sufficient to allow selection of the particular human serum albumin-related cDNA from a cDNA library containing many cDNA's unrelated to HSA.

To screen the above-described cDNA library, we pooled about 10,000 colonies from the library and grew up a culture (10 ml, $OD_{550}$ = 1.8) of the pooled colonies in 2YT medium supplemented with tetracycline (20 µg/ml) at 37°C. [J. H. Miller, Experiments In Molecular Genetics (Cold Spring Harbor Laboratory, Cold Spring Harbor, New York) (1972)]. Because plasmid pKT218 includes the gene coding for tetracycline resistance, E. coli HB101 which has been transformed with pKT218 (having that gene intact) will grow in medium containing that antibiotic to the exclusion of E. coli not so transformed. Therefore, growth in tetracycline-containing medium permits selection of hosts containing pKT218.

The culture was centrifuged (10 min, 5000 rpm) and resuspended in 1 ml 0.5 Tris-HCl (pH8) and lysozyme (1.6 mg/ml) and allowed to stand for 15 min in ice. The cold mixture was then combined with EDTA (to 8 mM) and allowed to remain in ice. It was then combined with a mixture (0.5 ml/ml) of 20% Triton X-100 (0.15 ml), 0.5 M EDTA (pH8) (3.75 ml), 1 M Tris-HCl (pH7.9) (1.5 ml), $H_2O$ (4.6 ml) and allowed to stand in ice for 15 min. The lysed cells were centrifuged in an Eppendorf centrifuge (5 min, 12000 rpm, 4°C) to remove the cellular debris from the lysed pKT218-based recombinant plasmids containing the inserted human fetal liver cDNA (supernatant).

The supernatant was applied to a low melting agarose gel (0.9%) and the upper portion of the "supercoiled" band (5000-7000 nucleotides in size) was removed from the gel by melting the agarose at 65°C. We expected that this band would contain all the pKT218 plasmids having cDNA inserts from the human fetal liver library of greater than about 750 base pairs. The separated agarose-containing pKT218-based recombinant plasmids were then employed to transform E. coli HB101 competent cells in ice (20 µl of gel/100 µl of cells) using conventional procedures [e.g., S. R. Kushner, in Genetic Engineering (H.W. Boyer ed.) p. 17 (1978)]

The cells were kept in ice for 45 min and then heated to 37°C for 5 min.  At that point 2YT broth (2 ml) was added and the cells incubated at 37°C with shaking for 45 min.  The cells were then plated on 2YT-containing agarose plates (containing 20 µg/ml tetracycline) and grown overnight at 37°C.

We next prepared three nitrocellulose filter replicates of the agarose plates and incubated them for 5 h at 37°C.  The colonies from two of the filters were transferred to 2YT-containing agarose (supplemented with 100 µg/ml chloramphenicol) and incubated for 15 h at 37°C. Chloramphenicol was employed to increase the copy number of the cDNA-containing plasmids in the host cells.  The cells were then lysed using the methods described in R. E. Thayer, "An Improved Method For Detecting Foreign DNA In Plasmids Of Escherichia Coli", Anal. Biochemistry, 98, pp. 60-63 (1979), except that the nitrocellulose filters were placed on the stack of Whatman No. 1 filter disks for 2 min instead of 1 min and the absorption and blotting procedure was repeated only once instead of two more times. The prepared filters were then dried and baked at 80°C for 2 h.  We obtained forty filters each containing 100-200 clones/filter from this procedure.

The filters were hybridized to a mouse serum albumin cDNA probe under non-stringent conditions because we believed there would be low homology between human serum albumin cDNA and mouse serum albumin cDNA.  We prepared the mouse serum albumin cDNA probe for this screening by culturing a host containing mouse serum albumin cDNA [D. Kioussis, supra] and excising the albumin insert from the plasmid by Hind III restriction.  That fragment was purfied on an 8% polyacrylamide gel and nick-translated in the presence all four $\alpha$-$^{32}$P-labelled nucleotides and polymerase I to a specific activity of $1.2 \times 10^8$ cpm/µg.  We then

prepared the filters for hybridization by incubating them for 12 h at 42°C in hybridization buffer [40% formamide, 0.6 M NaCl, 0.12 M Tris-HCl (pH8), 4 mM EDTA (hereinafter 5 X SET) and 10% Dextran sulfate, 0.1% SDS].

For hybridization two filters were placed in a polyethylene bag in the presence of 2 ml of fresh hybridization buffer and about 100 µg/ml of buffer of HinfI fragment of pBR322 and 1 x 10⁶ cpm of the above-isolated cDNA probe were added. Hybridization was continued at 42°C for 18 h. The filters were then washed for several hours (1 X SET, 0.5% SDS) at room temperature and then washed at 42°C for 20 min (1 X SET). The filters were then developed and the positions giving the strongest evidence of hybridization to the labelled probe selected for further investigation.

We used the twenty colonies, corresponding to the strongest hybridization-positive positions to prepare overnight cultures in 2YT broth at 37°C. The cultures were then streaked onto 2YT-agarose plates (containing 25 µg/ml tetracycline) and replicates made, as before, for hybridization. Eighteen of the twenty clones were again strongly positive to the labelled mouse serum albumin cDNA probe in our hybridization assay.

### ISOLATION OF HUMAN SERUM ALBUMIN RELATED cDNA

We prepared one ml cultures of the above-positive 18 colonies and centrifuged those in an Eppendorf tube (5 min, 12000 rpm). The cultures were resuspended in 70 µl STET buffer (8% sucrose, 50 mM Tris-HCl (pH8), 5mM EDTA, 5% Triton, H₂0), 5 µl Lysozyme (10 mg/ml) and allowed to stand at room temperature for 5 min. After boiling for 1 min, the lysed cells were centrifuged in an Eppendorf tube (5 min, 12000 rpm) and the supernatant combined with an equal volume of isopropanol. The isopropanol mixture was cooled to -20°C for 1 h and again centrifuged (4°C, 5 min, 12000 rpm). The pellet was then resuspended in 40 µl H₂0 for restriction analysis.

Each of the eighteen above-prepared mixtures contains a pKT218-human fetal liver cDNA recombinant that hybridizes strongly to our mouse albumin cDNA probe. Since this recombinant was prepared by dC/dG tailing and inserted at the PstI site of pKT218, the cDNA insert can be excised from each recombinant by PstI restriction. Accordingly, the eighteen mixtures were restricted with PstI under standard conditions and the fragments produced by the 18 positive cultures sized on a 1.5% agarose gel. The clones containing the ten larger fragments (about 1200-2250 base pairs each) were selected.

We used Southern hybridization to determine the orientation of the inserts in the 10 clones containing the largest inserts. For Southern hybridization, we used a 300-nucleotide cDNA sequence that we had isolated earlier, essentially as described above, from a similarly prepared human fetal liver cDNA library, except that it was characterized by short cDNA segments. By partial nucleotide sequencing, we had demonstrated that this sequence encoded amino acids 319 to 416 of human serum albumin. Accordingly, we used that HSA-related cDNA sequence to prepare a probe for the 5' end of HSA by restriction with PstI (HSA has a PstI site at amino acid 363). This restriction resulted in a probe spanning amino acids 319 to 363 of HSA. We then nick-translated that probe substantially as described by S.Y. Tsai et al., "Effect Of Estrogen On Gene Expression In The Chick Oviduct. Regulation Of The Ovomucoid Gene", Biochemistry, 17, pp. 5573-80 (1978).

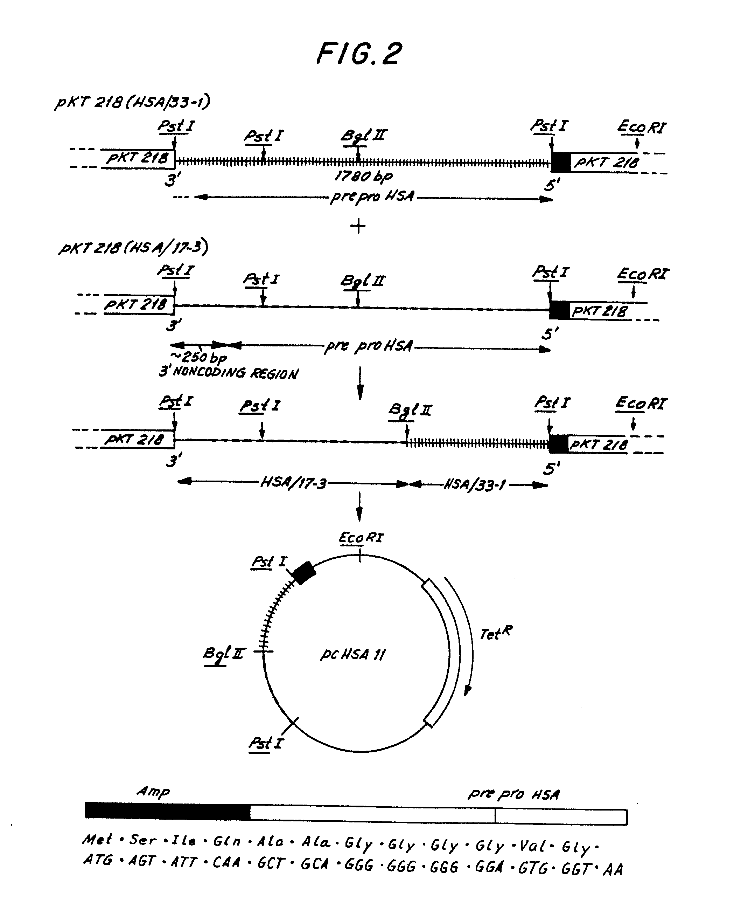

To prepare the ten mouse serum albumin cDNA hybridization positive and larger fragment containing clones for hybridization to the above HSA-related probe, we treated the clones with various restriction enzymes: PvuII, HindIII, PstI and PstI/HindIII. The particular fragments produced by these digests together with their hybridization to the 319-363 HSA probe enabled us to determine the orientation of the inserts and the location of the 5' end of each insert. Two of the ten clones pKT218(HSA/33-1) and pKT218(HSA/17-3), designated by their positions on the filters, had the 5' end in the correct orientation with respect to the direction of transcription of the gene coding for pencillinase into which the fragment had been inserted in pKT218. The structures of these two clones are depicted in Figure 2.

As can be seen in Figure 2, the insert of pKT218 (HSA/33-1) (hereinafter designated as pcHSA/33-1) comprised at least a part of what we later designated to be the nucleotide sequence coding for the presequence of human pro serum albumin and presumbably the entire coding sequence for what we have designated human proserum albumin. The insert of pKT218 (HSA/17-3), (hereinafter designated as pcHSA/17-3) comprised about 250 nucleotides from the 3' non-coding end of HSA and about the first 1600-1700 nucleotides of the coding sequence for HSA (i.e., lacking the coding sequence for about first the 30-35 amino acids). Accordingly, a combination of the two inserts (HSA/33-1 and HSA/17-3) affords a DNA sequence coding for substantial portions of the non-coding 3' end and at least part of presequence, as well as the entire coding sequence of human proserum albumin.

In order to construct this combination of HSA/33-1 and HSA/17-3, we restricted each clone with BglII and EcoRI and we ligated the shorter BglII-EcoRI fragment of pKT218 (HSA/33-1) to the longer BglII-EcoRI fragment of pKT218 (HSA/17-3). This new construction pKT218(HSA/33-1(BglII-EcoRI)- HSA/17-3(BglII-EcoRI) ("pcHSAll") is also depicted in Figure 2. Subsequent DNA sequencing revealed that this construction of pKT218(HSA/33-1(BglII-EcoRI)-HSA/17-3 (BglII-EcoRI) caused elimination of a 36 nucleotide BglII-BglII fragment from the DNA sequence encoding mature HSA. However, as demonstrated below, this deletion did not prevent that construction or other constructions also having that particular deletion from producing HSA-like polypeptides in hosts transformed with them.

## EXPRESSION OF HUMAN SERUM
## ALBUMIN-LIKE POLYPEPTIDES

We employed the recombinant DNA molecules described above: pKT218(HSA/33-1), pKT218(HSA/17-3) and pKT218(HSA/33-1(BglII-EcoRI) - HSA/17-3(BglII-EcoRI)), to transform E. coli HB101 using the earlier-described transformation procedures.

Cultures of these transformed organisms were then prepared and tested for the expression of human serum albumin-like polypeptides using a Broome-Gilbert assay [S. Broome and W. Gilbert, "Immunological Screening Method To Detect Specific Translation Products", Proc. Natl. Acad. Sci. USA, 74, pp. 2746-49 (1978)].

Anti-HSA IgG (available from Miles Laboratories) was purified using an HSA affinity column and labelled with $^{125}$I using standard techniques. Polyvinyl discs were then coated with anti-HSA IgG and the solid phase treated sequentially with the respective bacterial extracts lysed on the plates substantially as described by S. Broome and W. Gilbert, supra, and radioactively-labelled anti-HSA. Washing was carried out substantially as described by S. Broome and W. Gilbert, supra. The radioactivity of the solid phase was then monitored to determine the immune reaction.

In this assay, radioactively-labelled antibody will bind only to sites on the solid support where a polypeptide displaying an immunological property of HSA from the bacterial extract has been bound to the antibody of the solid phase. Therefore, labelled solid phase indicates the presence of HSA-like polypeptides in the extract.

The results of the radioimmunoassays were as follows:

| Extract | Assay |
|---|---|
| E. coli HB101(pKT218(HSA/33-1)) | + |
| E. coli HB101(pKT218(HSA/17-3)) | + |
| E. coli HB101(pKT218(HSA/33-1 (BglII-EcoRI)-HSA/17-3 (BglII-EcoRI))) | + |
| E. coli HB101(pKT218) | - (negative control) |
| HSA | + (positive control) |

## USE OF OTHER CLONING VECTORS AND HOST ORGANISM

It should also be understood that a wide variety of host/cloning vehicle combinations may be employed in cloning the HSA-related cDNA prepared in accordance with this invention. For example, useful cloning vehicles may consist of segments of chromosomal, non-chromosomal and synthetic DNA sequences, such as various known derivatives of SV40 and known bacterial plasmids, e.g., from E. coli including col El, pCR1, pBR322, pMB9 and their derivatives, wider host range plasmids, e.g., RP4, and phage DNAs, e.g., the numerous derivatives of phage λ, e.g., NM989, and other DNA phages, e.g., M13 and Filamenteous single-stranded DNA phages and vectors derived from combinations of plasmids and phage DNAs such as plasmids which have been modified to employ phage DNA or other expression control sequences or yeast plasmids, such as the 2 μ plasmid, or derivatives thereof. Useful hosts may include bacterial hosts such as strains of E. coli, such as E. coli HB 101, E. coli. χ1776, E. coli χ2282, E. coli MRCI and strains of Pseudomonas, Bacillus subtilis, Bacillus stearothermophilus and other E. coli, bacilli, yeasts and other fungi, animal or plant hosts such as animal (including human) or plant cells in culture or other hosts. Of course, not all host/vector combinations may be equally efficient. The particular selection of host/cloning vehicle combination may be made by those of skill in the art after due consideration of the principles set forth without departing from the scope of this invention. For example, the selection of an appropriate host is controlled by a number of factors recognized by the art. These include, for example, the compatibility with the chosen vector, the toxicity of proteins encoded by the hybrid plasmid, the ease of recovery of the desired protein, the expression characteristics of the vector and host, bio-safety and costs. A balance of these factors must be struck with the understanding that not all hosts may be equally effective for expression of a particular recombinant DNA molecule.

Furthermore, within each specific cloning vehicle various sites may be selected for insertion of the HSA-related DNA. These sites are usually designated by the restriction endonuclease which cuts them. For example, in pBR322 the PstI site is located in the gene for β-lactamase, between the nucleotide triplets that code for amino acids 181 and 182 of that protein. This site was empolyed by S. Nagata et al., supra, in its synthesis of polypeptides displaying an immunological or biological activity of leukocyte interferon. One of the two HindII endonuclease recognition sites is between the triplets coding for amino acids 101 and 102 and one of the several Taq sites at the triplet coding for amino acid 45 of β-lactamase in pBR322. In similar fashion, the EcoRI site and the PvuII site in pBR322 lie outside any coding region, e.g., the EcoRI site being located between the genes coding for resistance to tetracycline and ampicillin, respectively. These sites are well recognized by those of skill in the art. It is, of course, to be understood that a cloning vehicle useful in this invention need not have a restriction endonuclease site for insertion of the chosen DNA fragment. Instead, the vehicle could be cut and joined to the fragment by alternative means.

The specific vector or cloning vehicle, and in particular the site chosen therein for attachment of a selected DNA fragment to form a recombinant DNA molecule, is also determined by a variety of factors recognized by the art, e.g., the number of sites susceptible to a particular restriction enzyme, the size of the protein to be expressed, the susceptibility of the desired protein to proteolytic degradation by host cell enzymes, the contamination of the protein to be expressed by host cell proteins difficult to remove during purification, the expression characteristics, such as the location of start and stop codons relative to the vector sequences, and other factors recognized by those of skill in the art. The choice of a vector and an insertion site for a particular gene in that vector is determined by a balance of these factors, not all selections being equally effective for a given case.

Although several methods are known in the art for inserting foreign DNA into a cloning vehicle or vector to form a recombinant DNA molecule, the initial cloning method preferred in accordance with this invention was described above for pKT218. Of course, other known methods of inserting DNA sequences into cloning vehicles to form recombinant DNA molecules may be equally useful in this invention. These include, for example, dA-dT tailing, direct ligation, synthetic linkers, exonuclease and polymerase-linked repair reactions followed by ligation, or extensions of the DNA strands with DNA polymerase and an appropriate single-stranded template followed by ligation.

### DNA FRAGMENT MAPPING AND NUCLEOTIDE SEQUENCE DETERMINATION

Apart from their use to produce HSA-like polypeptides, the DNA sequences and recombinant DNA molecules of this invention are also useful in replication of specific nucleotide sequences containing all or a portion of the genome of HSA. HSA DNA prepared in this way may be used to determine the nucleotide sequence of portions of the genome, particularly those portions coding for the active parts of HSA. From those sequences, the structure of the polypeptides themselves may be determined. Knowledge of these sequences also permits modifications to be made in the recombinant DNA molecules of this invention to improve the yield of the polypeptide produced, to increase the activity of the polypeptides produced and to prepare derivatives of HSA.

Recombinant DNA molecule pKT218(HSA/33-1(BglII-EcoRI)-HSA/17-3(BglII-EcoRI)), since it contained substantial parts of the non-coding 3' sequence, as well as the complete coding sequence of what we have designated human proserum albumin (except for the missing 36 nucleotide BglII-BglII deletion) and at least a portion of its presequence may be used to determine the nucleotide sequence of the HSA genome over which it extends.

We constructed the physical map of the HSA/33-1 (BglII-EcoRI)-HSA/17-3(BglII-EcoRI) sequence by isolating the plasmid DNA, as before, and digesting the DNA with various restriction enzymes (New England Biolabs or BRL) in the recommended buffers by well-known procedures. The products of digestion were electrophoresed on 1% agarose gels. They were analyzed after visualization by staining with ethidium bromide and compared with detailed physical map of pBR322 [J. G. Sutcliffe, "Complete Nucleotide Sequence Of The Escherichia coli Plasmid pBR322", Cold Spring Harbor Symposium, 43, I, pp. 77-90 (1978)].

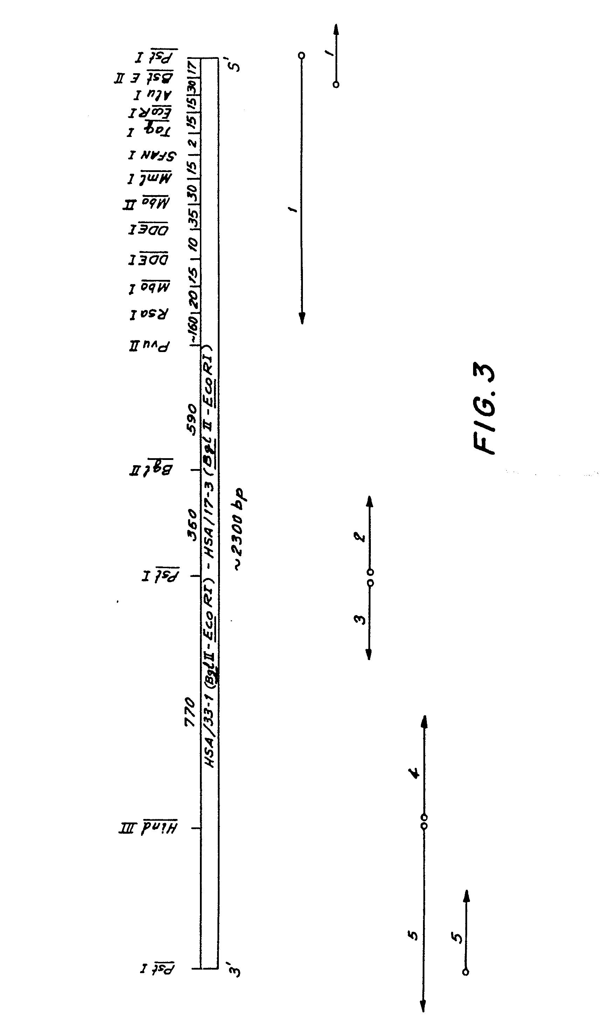

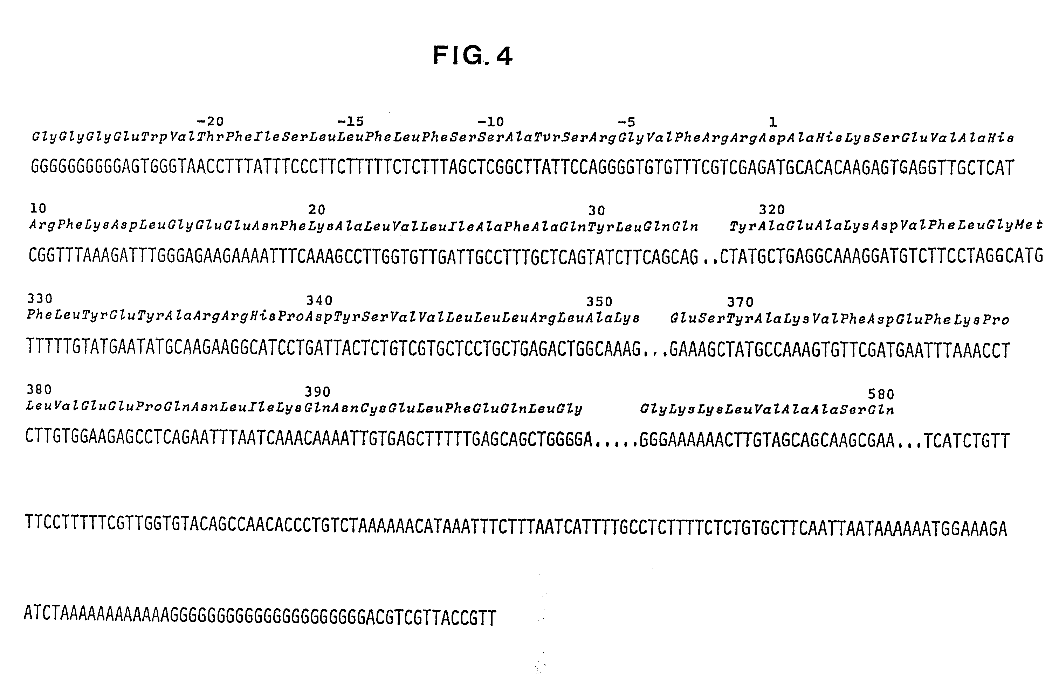

A partial restriction map of the HSA sequence was constructed on the basis of these digestion patterns. This map is depicted in Figure 3. We then refined the map by sequencing the DNA inserts, substantially as described by A. M. Maxam and W. Gilbert, "A New Method For Sequencing DNA", Proc. Natl. Acad. Sci. USA, 74, pp. 560-64 (1977). Figure 3 displays the various restriction fragments (the circles indicating the label and the arrow the direction of sequencing) and the sequencing strategy we employed to determine portions of the nucleotide sequence of pKT218 (HSA/33-1(BglII-EcoRI)-HSA/17-3(BglII-EcoRI)). The relevant portions of the nucleotide sequence we obtained for the insert of pKT218(HSA/33-1(BglII-EcoRI)-HSA/17-3(BglII-EcoRI)) are depicted in Figure 4. This sequence does not exclude the possibility that modifications to the sequence such as mutations, including single or multiple, base substitutions, deletions, insertions, or inversions have not already occurred or may not be employed subsequently to modify its expression or activity. It should also be understood that the remaining portions of the nucleotide sequence HSA/33-1(BglII-EcoRI)-HSA/17-3 (BglII-EcoRI) may be determined by similar techniques.

By comparing the polypeptides coded for by the various regions of the HSA/33-1(BglII-EcoRI)-HSA/17-3 (BglII-EcoRI) nucleotide sequence which we determined with the 585 amino acids determined for natural HSA [B. Meloun, supra.], it appears that the DNA sequence of that insert codes for sixteen amino acids (designated as amino acids -7 to -22 in Figure 4) of a sequence which because of its hydrophobic character we have assigned as at least part of a putative presequence of human serum albumin and the entire amino acid sequence of a polypeptide having 591 amino acids. On the basis of the reported amino acid sequence of natural HSA [Meloun, supra], we have designated this larger protein human proserum albumin (it has six amino acids (designated as amino acids -1 to -6 in Figure 4) between the presequence and the amino acid sequence reported for natural human serum albumin).

It also appears (from the portions of the nucleotide sequence that we have determined) that the coding sequence of HSA/33-1(BglII-EcoRI)-HSA/17-3(BglII-EcoRI) is out of phase by a single nucleotide, as compared with the coding sequence of the gene coding for penicillinase resistance into which we have inserted it in pBR322. However, as a result of perhaps some internal start, hosts transformed with the hybrid gene (even out of phase) in pKT218 still produce HSA-like products.

It should of course be understood that the coding sequence of human proserum albumin, as isolated and described by us, may be employed in other constructions to prepare plasmids where the coding sequence is in phase with the penicillinase gene or to prepare other plasmids to improve the yield and activity of HSA-like polypeptides in accordance with this invention.

-20-

IMPROVING THE YIELD AND ACTIVITY
OF HSA-LIKE POLYPEPTIDES
PRODUCED IN ACCORDANCE WITH THIS INVENTION

The level of production of a protein in a host is governed by two major factors: the number of copies of its gene within the cell and the efficiency with which those gene copies are transcribed and translated. Efficiency of transcription and translation (which together comprise expression) is in turn dependent upon nucleotide sequences, normally situated ahead of the desired coding sequence. These nucleotide sequences or expression control sequences define, inter alia, the location at which RNA polymerase interacts to initiate transcription (the promoter sequence) and at which ribosomes bind and interact with the mRNA (the product of transcription) to initiate translation. Not all such expression control sequences function with equal efficiency. It is thus of advantage to separate the specific coding sequences for a desired protein from their adjacent nucleotide sequences and to fuse them instead to other known expression control sequences so as to favor higher levels of expression or perhaps higher levels of secretion and maturation from the host cell. This having been achieved, the newly engineered DNA fragment may be inserted into a multicopy plasmid or a bacteriophage derivative in order to increase the number of gene copies within the cell and thereby further to improve the yield of expressed protein.

Several expression control sequences may be employed as described above. These include the operator, promoter and ribosome binding and interaction sequences (including sequences such as the Shine-Dalgarno sequences) of the lactose operon of E. coli ("the lac system"), the corresponding sequences of the tryptophan synthetase system of E. coli ("the trp system"), the β-lac system, the major operator and promoter regions of phage λ ($O_L P_L$ and $O_R P_R^I$), the control region of Filamenteous single stranded DNA phages, or other sequences which control the expression of genes of prokaryotic or eukaryotic cells and their viruses and various combinations of those sequences, e.g. the TAC system or the TRC system. Therefore, to improve the production of a particular polypeptide in an appropriate host, the gene coding for that polypeptide may be selected as before and removed from the recombinant DNA molecule containing it and the gene reinserted into a another cloning vehicle or expression vector closer or in a more appropriate relationship* to its former expression control sequence or under the control of one of the above improved expression control sequences. Such methods are known in the art.

Prior to or subsequent to such constructions, the DNA sequences encoding the HSA-like polypeptides may also be combined with various signal sequences, both prokaryotic or eukaryotic in origin, or combinations thereof, so as to permit the HSA-like polypeptide to be secreted from the host cell preferably with cleavage of the signal sequence during secretion. Such methods are described, for example, in Villa-Komaroff et al., supra, and Talmadge et al., supra.

Further increases in the cellular yield of the desired products depend upon an increase in the number of genes that can be utilized in the cell. This may be achieved, for illustration purposes, by insertion of recombinant DNA molecules engineered in the way described previously into the temperate bacteriophage λ (NM989), most simply by digestion of the plasmid with a restriction enzyme, to give a linear molecule which is then mixed with a restricted phage λ cloning vehicle [e.g., of the type

---

* As used herein "relationship" may encompass many factors, e.g., the distance separating the expression enhancing and promoting regions of the recombinant DNA molecule and the inserted DNA sequence, the transcription and translation characteristics of the inserted DNA sequence or other sequences in the vector itself, the particular nucleotide sequence of the inserted DNA sequence and other sequences of the vector and the particular characteristics of the expression enhancing and promoting regions of the vector.

described by N. E. Murray et al., "Lambdoid Phages That Simplify The Recovery Of In Vitro Recombinants", <u>Molec. gen. Genet.</u> 150, pp. 53-61 (1977) and N. E. Murray et al., "Molecular Cloning Of The DNA Ligase Gene From Bacterio-phage T4", <u>J. Mol. Biol.</u>, 132, pp. 493-505 (1979)] and the recombinant DNA molecule recircularized by incubation with DNA ligase. The desired recombinant phage is then selected as before and used to lysogenise a host strain of <u>E. coli</u>.

Particularly useful λ cloning vehicles contain a temperature-sensitive mutation in the repressor gene <u>cI</u> and suppressible mutations in gene <u>S</u>, the product of which is necessary for lysis of the host cell, and gene <u>E</u>, the product which is the major capsid protein of the virus. With this system the lysogenic cells are grown at 32°C and then heated to 45°C to induce excision of the prophage. Prolonged growth at 37°C leads to high levels of production of the protein, which is retained within the cells, since these are not lysed by phage gene products in the normal way, and since the phage gene insert is not encapsidated it remains available for further transcription. Artificial lysis of the cells then releases the desired product in high yield.

As another illustration, the coding sequence of a gene coding for an HSA-like polypeptide of this invention could be inserted into an expression vector under $P_L$ control, substantially as described in H. Küpper et al., "Cloning Of cDNA Of Major Antigen Of Foot And Mouth Disease Virus And Expression In <u>E. coli</u>", <u>Nature</u>, 289, pp. 555-59 (1981) for the genes coding for polypeptides displaying the antigenicity of FMDV.

In addition, it should be understood that the yield of HSA-like polypeptides prepared in accordance with this invention may also be improved by substituting different codons for some or all of the codons of the present DNA sequences. These substituted codons would code for amino acids similar or identical to those coded for by the codons replaced, but would be more favorably expressed in the particular host chosen for HSA production.

Finally, the activity of the polypeptides produced by the recombinant DNA molecules of this invention may be improved by fragmenting, modifying or derivatizing the DNA sequences or polypeptides of this invention by well-known means, without departing from the scope of this invention.

As one example of isolating a DNA sequence in accordance with this invention and joining it to an expression control sequence, we isolated HSA/33-1(BglII-EcoRI)-HSA/17-3(BglII-EcoRI) from the above-described vector and joined it downstream of the TAC promoter in another expression vector.

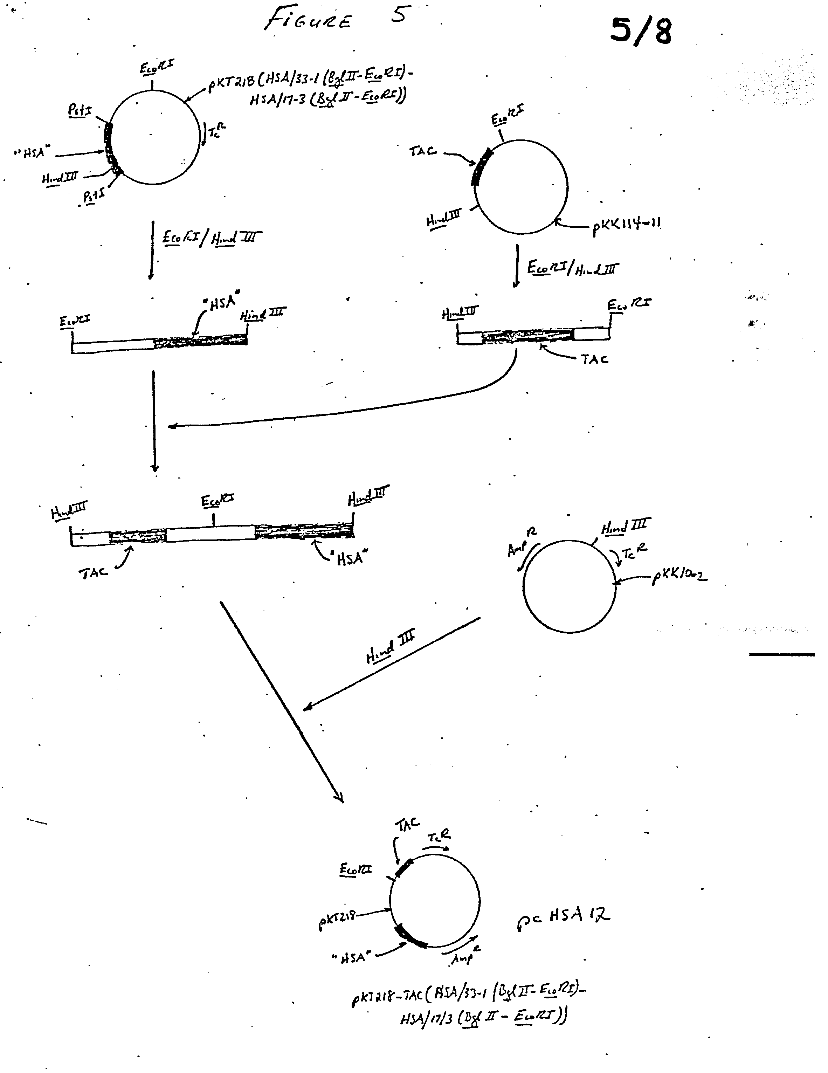

To make this construction we restricted pKT218 (HSA/33-1(BglII-EcoRI)-HSA/17-3(BglII-EcoRI)), isolated as described previously, with EcoRI and HindIII as depicted in Figure 5. We then combined the EcoRI-HindIII fragment containing the HSA coding sequences at its EcoRI terminus with a EcoRI-HindIII fragment from pKK114-11 (a gift of Jürgen Brosius), containing the TAC expression control sequence. The resulting fragment (having two HindIII termini) was then recircularized by combination with the HindIII fragment of pKK10-2 (a derivative of pBR322 and a gift of Jürgen Brosius). We designated this recombinant DNA molecule as pKT218-TAC(HSA/33-1(BglII-EcoRI)-HSA/17-3 (BglII-EcoRI)) or pcHSA12. When we transformed E. coli HB101 with this recombinant DNA molecule, we observed a 20-30 times higher production of HSA-like polypeptides.

To determine the location of HSA-like polypeptides produced in host transformed with pKT218-TAC(HSA/33-1 (BglII-EcoRI)-HSA/17-3(BglII-EcoRI)). We incubated 10 ml sulfur free-medium [R.B. Roberts et al., "Studies Of Biosynthesis In E.coli", Kirby Lithographic Co., Inc., p. 5 (1957)] with 2 ml cultures of E.coli HB101(pKT218-TAC(HSA/ 33-1(BglII-EcoRI)-HSA/17-3 (BglII-EcoRI))) until OD = 0.2. We then induced the cultures with 5 mM IPTG and continued incubation to OD = 0.5. The cultures were then labelled with $^{35}S$-$H_2SO_4$ (5 $mc_i$, 30 min, 37°C) and centrifuged (5 min, 12000 rpm). The pellets were resuspended in Tris-HCl (pH8), 25% sucrose, 25 µl lysozyme (10 mg/ml) and ice for 15 min and again centrifuged (5 min, 12000 rpm) and the supernatant (containing the periplasmic protein fraction of the transformed cells) removed. The pellets (spheroplasts) were resuspended in 225 µl Tris-HCl (pH8), 25% sucrose and 725 µl Triton-lysis mix [0.15M Tris-HCl (pH8), 0.2 M EDTA, 2% Triton X100] added. The mixture was allowed to stand in ice for 15 min and then centrifuged (5 min, 12000 rpm, 4°C) to remove the cellular debris (the super-naturant containing the intracellular protein fraction of the transformed host cells).

We then analyzed the periplasmic fraction-containing supernatant and the intracellular fraction-containing supernatant for the presence of HSA-like polypeptides. We added 1000-fold excesses of anti-HSA to both fractions and allowed them to stand for 1 hour at 37°C. We then added 300 µl Sepharose Protein A (30 mg/ml), allowed the mixtures to stand at room temperature for 1 hour and centrifuged them (5 min, 12000 rpm). The pellets were resuspended in 1 ml NET-NT buffer [0.65 M NaCl, 5mM EDTA, 50mM Tris-HCl (pH7.5), 1% Triton X100], centrifuged (5 min, 12000 rpm), resuspended in the same buffer, centrifuged (5 min, 12000 rpm) and resuspended in the same buffer, except that the buffer was 0.15 M NaCl not 0.65 M. We repeated this process of resuspension and centrifugation twice and then suspended the final pellets in 30 µl Lämmlè buffer. Analysis of the gel demonstrated the presence of about 50% of the HSA-like polypeptides produced by the transformed host in the periplasmic space fraction and about 50% of the HSA-like polypeptides in the intracellular fraction.

The HSA-like polypeptide produced in this host appeared to be smaller than natural HSA. This observed difference in size to some extent may be explained by the missing 36 nucleotides in the HSA coding sequence. However, the size difference is greater than that which could be accounted for by the 12 amino acids encoded by those missing 36 nucleotides. The remainder of the size difference may therefore be explained by production of the protein from an internal start, degradation of protein or perhaps incorrect processing. In any event the polypeptides displayed an immunological activity consistent with natural HSA.

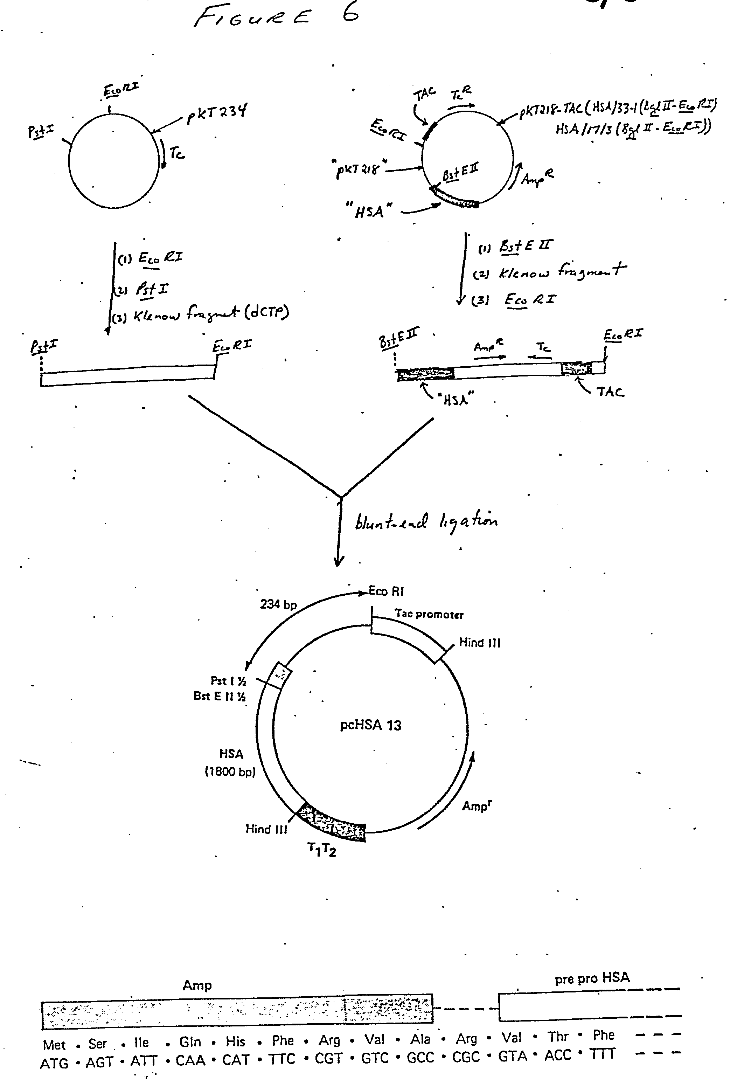

Since the above construction also had the HSA coding sequence out of phase with the gene coding for penicillinase by a single nucleotide, we used it to prepare a construction having the correct reading frame between the gene coding for penicillinase and the HSA-related DNA fragment. As shown in Figure 6, we restricted the construction with BstEII and filled in the overlapping end of the fragment with a Klenow fragment. The DNA was then further restricted with EcoRI and the larger BstEII-EcoRI fragment isolated. We combined this fragment with a fragment that we isolated from plasmid pKT234 (K. Talmadge et al., supra) that had been restricted with EcoRI and PstI and filled in (Klenow fragment, dCTP only). The resulting construction has one nucleotide less between the portion of the gene coding for penicillinase and the HSA-related coding sequence. We designated this hybrid molecule pcHSA13. Analysis of the HSA-like polypeptides produced by E.coli HB101 transformed with this modified hybrid gene demonstrated that about the same amount of HSA-like polypeptides were produced per cell as in the host transformed with pKT218-TAC(HSA/33-1(BglII-EcoRI)-HSA/17-3(BglII-EcoRI)). The level of expression is about $4.5 \times 10^4$ molecule/cell. Moreover, the product had about the same size on an SDS/acrylamide gel as natural HSA. This was unexpected because the product was derived from a DNA sequence having the above described 36 nucleotide deletion.

We have also prepared other expression systems characterized by DNA sequences encoding HSA-like polypeptides. These constructions were designed to produce a DNA sequence encoding HSA-like polypeptides without the 36-nucleotide BglII-BglII deletion. They were also designed to employ various combinations of expression control sequences and signal sequences before the HSA coding sequence.

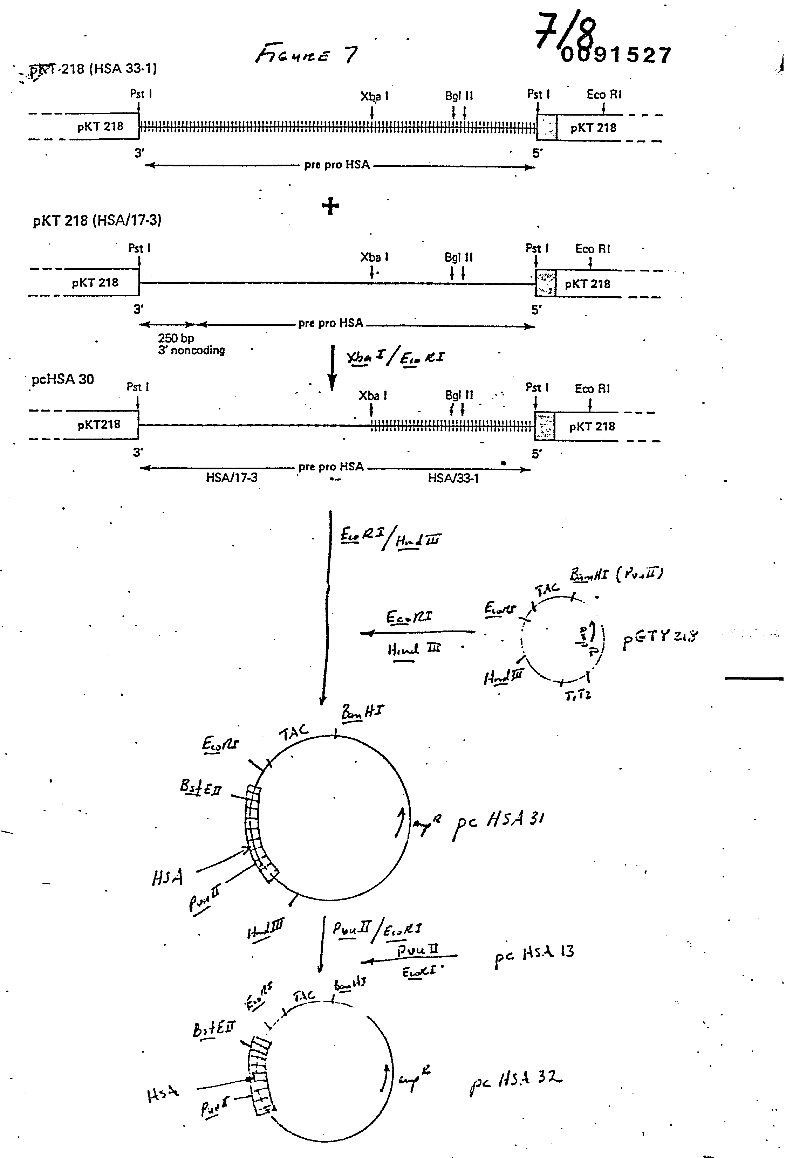

Referring now to Figure 7, we have depicted therein the construction of pcHSA30 from pKT218(HSA/33-1) and pKT218(HSA/17-3) by XbaI/EcoRI digestion and religation. This process avoids the 36 nucleotide deletion occasioned by BglII restriction of the HSA coding sequences. We then employed pcHSA30 to produce pcHSA31 and pcHSA32. These recombinant DNA molecules have a TAC expression control sequence derived from pGFY218 (a gift of Jürgen Brosius). We transformed E.coli W3110I$^Q$ with pcHSA32. E.coli W3110I$^Q$ is a strain having a mutation in its i gene that represses expression of DNA sequences under the control of the TAC expression control sequence (e.g., the HSA coding sequence of pcHSA32) until induction by IPTG. Upon induction with IPTG, the transformed strain produced about 8000 molecules/ cell of an HSA-like polypeptide. This polypeptide had about the same size (SDS/acrylamide gel) as natural HSA.

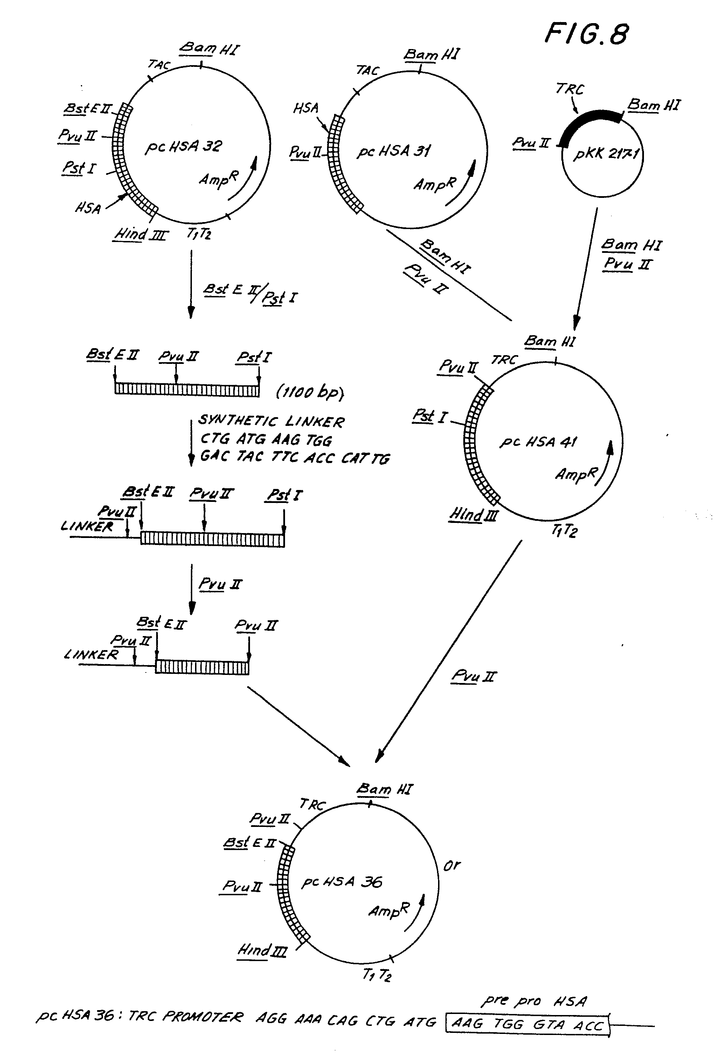

We also employed pcHSA31 and pcHSA32 to construct a recombinant DNA molecule (pcHSA36) wherein the HSA coding sequence is under the control of the TRC expression control sequence. The TRC sequence (a gift of Jürgen Brosius) is a derivative of the previously described TAC system. We have depicted the construction of pcHSA36 in Figure 8.

We employed pcHSA36 to transform E.coli W3110I$^Q$. After induction as before, the transformed strain produced large amounts of a protein of about the same size (SDS/acrylamide gel) as natural HSA. Moreover, this protein appears primarily as a visible single band after antibody precipitation. Accordingly, it is believed that this construction, the most preferred construction of our invention, produces large amounts of an HSA-like polypeptide. However, the transformed host dies shortly after induction. We believe that this may be due to the toxic effects of the large amounts of HSA-like polypeptides produced by the host. Other strains and growth or induction conditions should permit maintenance of the strains transformed with pcHSA36 and the production of large amounts of the desired product.

Microorganims, DNA sequences and recombinant DNA molecules prepared by the processes described herein are exemplified by cultures deposited in the culture collection of the American Type Culture Collection in Rockville, Maryland on December 14, 1981, and identified there as HSA-A and assigned ATCC accession number 39026.

     A: E.coli HB101(pKT218(HSA/33-1(BglII-EcoRI)-HSA/17-3(BglII-EcoRI)))

Another microorganism, DNA sequence and recombinant DNA molecule of this invention was deposited in the culture collection of the American Type Culture Collection in Rockville, Maryland on December 9th, 1982 and identified there as HSA-B and assigned ATCC accession number 39253 .

     B: E.coli W3110I$^Q$ (pcHSA 36)

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than by the specific embodiments which have been presented hereinbefore by way of example.

CLAIMS:

1. A DNA sequence characterized in that at least a portion thereof codes for a polypeptide displaying an immunological or biological activity of human serum albumin and being selected from the group consisting of (a) HSA/33-1, HSA/17-3, HSA/33-1(BglII-EcoRI)-HSA/17-3(BglII-EcoRI), HSA/33-1(XbaI-EcoRI)-HSA/17-3(XbaI-EcoRI) (b) DNA sequences which hybridize to any of the foregoing DNA sequences, (c) DNA sequences, from whatever source obtained, including natural, synthetic or semisynthetic sources, related by mutation, including single or multiple, base substitutions, deletions, insertions and inversions to any of the foregoing DNA sequences, and (d) DNA sequences comprising sequences of codons which code for a polypeptide containing an amino acid sequence similar to those coded for by codons of any of the foregoing DNA sequences, said DNA sequences b through d coding for a polypeptide displaying an immunological or biological activity of human serum albumin.

2. A recombinant DNA molecule comprising a DNA sequence according to claim 1.

3. A recombinant DNA molecule according to claim 2, wherein said DNA sequence is operatively linked to an expression control sequence.

4. A recombinant DNA molecule according to claim 3 wherein the expression control sequence is selected from the group consisting of the E. coli lac system, the E. coli trp system, the E.coli β-lac system, the TAC system, the TRC system, the major operator and promoter regions of phage λ, the control region of Filamenteous single-stranded DNA phages, other sequences which control the expression of genes of prokaryotic or eukaryotic cells and their viruses and combinations thereof.

5. A recombinant DNA molecule according to claim 4, selected from the group consisting of pKT218 (HSA/33-1), pKT218(HSA/17-3), pKT218(HSA/33-1(BglII-EcoRI)-HSA/17-3(BglII-EcoRI)), pKT218-TAC(HSA/33-1(BglII-EcoRI)-HSA/17-3(BglII-EcoRI)) pcHSA32 and pcHSA36.

6. A host transformed with at least one recombinant DNA molecule according to claim 4 or 5.

7. A transformed host according to claim 6 wherein the host transformed is selected from the group consisting of strains of E. coli, Pseudomonas, Bacillus subtilis, Bacillus stearothermophilus, other bacilli, yeasts, other fungi, animal and plant hosts and human tissue cells.

8. A transformed host according to claim 6 or 7, selected from the group consisting of E.coli HB101(pKT218 (HSA/33-1)), E.coli HB101(pKT218(HSA/17-3)), E.coli HB101(pKT218(HSA/33-1(BglII-EcoRI)-HSA/17-3(BglII-EcoRI))), E.coli W3110I$^{Q}$ (pcHSA32) and E.coli W3110I$^{Q}$ (pcHSA36).

9. A polypeptide or fragment or derivative thereof displaying an immunological or biological activity of human serum albumin and produced by a transformed host according to any one of claims 6 to 8.

10. A polypeptide characterized in that at least a portion of it is coded for by a DNA sequence according to claim 1.

11. A method for producing a recombinant DNA molecule comprising the step of introducing into a cloning vehicle a DNA sequence according to claim 1.

12. A method according to claim 11 further comprising the additional step of introducing into said cloning vehicle an expression control sequence, said expression control sequence being introduced into said cloning vehicle so as to control and to regulate the expression of said DNA sequence.

13. A method for transforming a host comprising the step of introducing into a host a recombinant DNA molecule according to claim 4 or 5.

14. A method for producing a polypeptide displaying an immunological or biological activity of human serum albumin comprising the steps of transforming an appropriate host with a recombinant DNA molecule according to claim 4 or 5; culturing said host; and collecting said polypeptide.

15. The method according to claim 14, characterized in that the host transformed is selected from the group consisting of strains of <u>E. coli</u>, <u>Pseudomonas</u>, <u>Bacillus subtilis</u>, <u>Bacillus stearothermophilus</u>, other bacilli, yeasts, fungi, animal or plant hosts, and human tissue cells.

16. A method for producing a polypeptide displaying an immunological or biological activity of human serum albumin comprising the steps of culturing a host transformed by a recombinant DNA molecule according to claim 4 or 5 and collecting said polypeptide.

17. A process for selecting a DNA sequence coding for a polypeptide displaying an immunological or biological activity of human serum albumin from a group of DNA sequences, comprising the step of screening the DNA sequences of the group to determine which hybridize to at least one of the DNA sequences according to claim 1.

18. The process of claim 17 wherein the DNA sequence screened is selected from the group consisting of DNA sequences from natural sources, synthetic DNA sequences, DNA sequences from recombinant DNA molecules and DNA sequences which are a combination of any of the foregoing DNA sequences.

19. A pharmaceutically-acceptable composition comprising a polypeptide selected from the group consisting of the polypeptides of claim 9 or 10 and the polypeptides produced by the methods of any one of claims 14 to 16.

20. A method for treating humans comprising the step of treating them in a pharmaceutically-acceptable manner with a composition according to claim 19.

HUMAN FETAL LIVER cDNA LIBRARY

    (1) CULTURE
    (2) POOL
    (3) ISOLATE PLASMIDS OF 5000-7000 NUCLEOTIDES

COLLECTION OF pKT 218 (cDNA)

    TRANSFORM
    E. coli HB 101

COLLECTION OF E. coli HB 101 (pKT 218 [cDNA])

    (1) CULTURE
    (2) REPLICATE ON TO FILTERS
    (3) CHLORAMPHENICOL
    (4) LYSIS

40 FILTERS (100 - 200 CLONES/FILTER)

    HYBRIDIZATION SELECTION
    WITH MOUSE SERUM ALBUMIN
    cDNA PROBE

18 CLONES

    SELECTION
    (1) SIZE OF INSERT
    (2) ORIENTATION OF INSERT

pKT 218 (HSA/33-1) + pKT 218 (HSA/17-3)

FIG. 1

FIG.2

FIG.3

# FIG. 4

```
           -20              -15              -10               -5               1
GlyGlyGlyGluTrpValThrPheIleSerLeuLeuPheLeuPheSerSerAlaTyrSerArgGlyValPheArgArgAspAlaHisLysSerGluValAlaHis
GGGGGGGGGGGAGTGGGTAACCTTTATTTCCCTTCTTTTTCTCTTTAGCTCGGCTTATTCCAGGGGTGTGTTTCGTCGAGATGCACACAAGAGTGAGGTTGCTCAT

10                         20                                30                        320
ArgPheLysAspLeuGlyGluGluAsnPheLysAlaLeuValLeuIleAlaPheAlaGlnTyrLeuGlnGln   TyrAlaGluAlaLysAspValPheLeuGlyMet
CGGTTTAAAGATTTGGGAGAAGAAAATTTCAAAGCCTTGGTGTTGATTGCCTTTGCTCAGTATCTTCAGCAG..CTATGCTGAGGCAAAGGATGTCTTCCTAGGCATG

330                        340                               350                       370
PheLeuTyrGluTyrAlaArgArgHisProAspTyrSerValValLeuLeuLeuArgLeuAlaLys   GluSerTyrAlaLysValPheAspGluPheLysPro
TTTTTGTATGAATATGCAAGAAGGCATCCTGATTACTCTGTCGTGCTCCTGCTGAGACTGGCAAAG...GAAAGCTATGCCAAAGTGTTCGATGAATTTAAACCT

380                        390                                                  580
LeuValGluGluProGlnAsnLeuIleLysGlnAsnCysGluLeuPheGluGlnLeuGly   GlyLysLysLeuValAlaAlaSerGln
CTTGTGGAAGAGCCTCAGAATTTAATCAAACAAAATTGTGAGCTTTTTGAGCAGCTGGGGA.....GGGAAAAAACTTGTAGCAGCAAGCGAA...TCATCTGTT


TTCCTTTTTCGTTGGTGTACAGCCAACACCCTGTCTAAAAAACATAAATTTCTTTAATCATTTTGCCTCTTTTCTCTGTGCTTCAATTAATAAAAAATGGAAAGA


ATCTAAAAAAAAAAAAGGGGGGGGGGGGGGGGGGGGGGGGGGGACGTCGTTACCGTT
```

FIGURE 5

# FIGURE 6

blunt-end ligation

pcHSA 13

234 bp — Eco RI

Tac promoter

Hind III

Pst I ½
Bst E II ½

HSA
(1800 bp)

Hind III

$T_1T_2$

Amp$^r$

| Amp | | | | | | | | | pre pro HSA | | |
|---|---|---|---|---|---|---|---|---|---|---|---|
| Met | Ser | Ile | Gln | His | Phe | Arg | Val | Ala | Arg | Val | Thr | Phe — — — |
| ATG | AGT | ATT | CAA | CAT | TTC | CGT | GTC | GCC | CGC | GTA | ACC | TTT — — — |

Figure 7

7/8
0091527

FIG.8